# EUROPEAN PATENT APPLICATION

(11) **EP 1 728 510 A2**
(43) Date of publication of application: **06.12.2006**
(21) Application number: 06012623.2
(22) Date of filing: 13.11.2000
(51) Int. Cl.: A61K 31/365, A61K 31/341, A61K 45/06, G01N 33/50, A61K 31/00

(54) **Treating cancer by increasing intracellular malonyl coA levels**

(30) Priority: 12.11.1999 US 164768 P; 12.11.1999 US 164765 P
(62) Divisional of application: 00978553.6
(71) Applicant: The Johns Hopkins University, School of Medicine, Office of Technology Licencing, Baltimore, MD 212002 (US)
(72) Inventor: Pizer, Ellen S., Maryland 21029 (US); Townsend, Craig A., Maryland 21212 (US); Kuhajda, Francis P., Maryland 21209 (US)
(74) Representative: Dean, John Paul

(57) **Abstract**

This invention describes a method to kill cancer cells by acute elevation of cellular malonyl Coenzyme A (Malonyl CoA) which leads to apoptosis. Elevation of malonyl CoA may be induced by inhibition of fatty acid synthase (FAS), or by other manipulation of fatty acid metabolism tht does not involve inhibition of FAS. Alternatively, growth of tumor cells may be induced by a combination of effects including FAS inhibition in conjunction with other interventions which affect fatty acid metabolism, including inhibition of camitine palmitoyltransferase-1 (CPT-1). Any combination of drugs which produces an analogous physiologic effect(s) may be expected to lead to the same effect on susceptible tumor cells. For example, combination therapy with drug(s) that inhibit the fatty acid synthesis by inhibiting acetyl CoA carboxylase (the first enzyme in the fatty acid synthesis pathway) and drug(s) that inhibit CPT-1 may be expected to induce apoptosis in tumor cells. Therefore, this invention encompasses any method to systemically modify fatty acid metabolism in cancer cells including but not limited to direct inhibition of CPT-1 through small molecule inhibitors such as etomoxir, as well as inhibition of CPT-1 incidental to increasing the level of malonyl CoA in cancer cells.

## Description

### Review of Related Art

A number of studies have demonstrated surprisingly high levels of fatty acid synthase expression (FAS, E.C. 2.3.1.85) in virulent human breast cancer (Alo, P. L., Visca, P., Marci, A., Mangoni, A., Botti, C., and Di Tondo, U. Expression of fatty acid synthase (FAS0 as a predictor of recurrence in stage I breast carcinoma patients., Cancer. 77: 474-482, 1996; Jensen, V., Ladekarl, M., Holm-Nielsen, P., Melsen, F., and Soerensen, F. B. The prognostic value of oncogenic antigen 519 (OA-519) expression and proliferative activity detected by antibody MIB-1 in node-negative breast cancer., Journal of Pathology. 176: 343-352, 1995), as well as other cancers (Rashid, A., Pizer, E. S., Moga, M., Milgraum, L. Z., Zahurak, M., Pasternack, G. R., Kuhajda, F. P., and Hamilton, S. R. Elevated expression of fatty acid synthase and fatty acid synthetic activity in colorectal neoplasia., American Journal of Pathology. 150: 201-208, 1997; Pizer, E., Lax, S., Kuhajda, F., Pasternack, G., and Kurman, R. Fatty acid synthase expression in endometrial carcinoma: correlation with cell proliferation and hormone receptors., Cancer. 83: 528-537, 1998). FAS expression has also been identified in intraductal and lobular *in situ* breast carcinoma; lesions associated with increased risk for the development of infiltrating breast cancer (Milgraum, L. Z., Witters, L. A., Pasternack, G. R., and Kuhajda, F. P. Enzymes of the fatty acid synthesis pathway are highly expressed in in situ breast carcinoma., Clinical Cancer Research. 3: 2115-2120, 1997). FAS is the principal synthetic enzyme of fatty acid synthesis (FA synthesis) which catalyzes the NADPH dependent condensation of malonyl-CoA and acetyl-CoA to produce predominantly the 16-carbon saturated free fatty acid, palmitate (Wakil, S. Fatty acid synthase, a proficient multifunctional enzyme., Biochemistry. 28: 4523-4530, 1989). *Ex vivo* measurements in tumor tissue have revealed high levels of both FAS and FA synthesis indicating that the entire genetic program is highly active consisting of some 25 enzymes from hexokinase to FAS.

Cultured human cancer cells treated with inhibitors of FAS, including the fungal product, cerulenin, and the novel compound, C75, demonstrated a rapid decline in FA synthesis, with subsequent reduction of DNA synthesis and cell cycle arrest, culminating in apoptosis (Pizer, E. S., Jackisch, C., Wood, F. D., Pasternack, G. R., Davidson, N. E., and Kuhajda, F. Inhibition of fatty acid synthesis induces programmed cell death in human breast cancer cells., Cancer Research. 56: 2745-2747, 1996, Pizer, E. S., Chrest, F. J., DiGiuseppe, J. A., and Han, W. F. Pharmacological inhibitors of mammalian fatty acid synthase suppress DNA replication and induce apoptosis in tumor cell lines., Cancer Research. 58: 4611-4615, 1998). Pharmacological inhibition of mammalian fatty acid synthase activity lead to inhibition of DNA replication within about 90 minutes of drug application. These findings suggested a vital biochemical link between FA synthesis and cancer cell growth. While generating a great deal of interest, the question of how inhibition of fatty acid synthase triggered this phenomenon remained unknown. Importantly, these effects occurred despite the presence of exogenous fatty acids in the culture medium derived from fetal bovine serum. While it has been possible to rescue the cytotoxic effect of cerulenin on certain cells in fatty acid-free culture conditions by the addition of exogenous palmitate, most cancer cells were not rescued from FA synthesis inhibition by the pathway endproduct (data not shown) (Pizer, E. S., Wood, F. D., Pasternack, G. R., and Kuhajda, F. P. Fatty acid synthase (FAS): A target for cytotoxic antimetabolities in HL60 promyelocytic leukemia cells., Cancer Research. 1996: 745-751, 1996). Thus, it has been unresolved whether the cytotoxic effect of FA synthesis inhibition on most cancer cells resulted from end product starvation, or from some other biochemical mechanism.

### Summary of the Invention

This invention describes a method to kill cancer cells by acute elevation of cellular malonyl Coenzyme A (Malonyl CoA) which leads to apoptosis. Elevation of malonyl CoA induced by inhibition of fatty acid synthase (FAS), is correlated with both inhibition of fatty acid synthesis and also with inhibition of carnitine palmitoyltransferase-1 (CPT-1). Any combination of drugs which produces an analogous physiologic effect may be expected to lead to the same effect on susceptible tumor cells. For example, combination therapy with drug(s) that inhibit the fatty acid synthesis by inhibiting acetyl CoA carboxylase (the first enzyme in the fatty acid synthesis pathway) and drug(s) that inhibit CPT-1 may be expected to induce apoptosis in tumor cells. Therefore, this invention encompasses any method to systemically inhibit the activity of CPT-1 in cancer cells including but not limited to direct inhibition of CPT-1 through small molecule inhibitors such as etomoxir, as well as inhibition of CPT-1 incidental to increasing the level of malonyl CoA in cancer cells.

This therapeutic strategy will lead to novel chemotherapeutic agents for a wide variety of human cancers. In addition, as this is a novel pathway leading to apoptosis which is not shared by other cancer drugs, it may be anticipated that induction of high levels of malonyl CoA and/or CPT-1 inhibition may potentiate other commonly utilized cancer therapeutic agents.

In one embodiment, this invention provides a method for inhibiting growth of tumor cells in an organism by administering to the organism a composition which causes a rise in intracellular malonyl CoA in tumor cells of the organism. Preferably, the intracellular malonyl CoA in at least the tumor cells of the organism rises abruptly (i.e., acutely or sharply), and more preferably, the intracellular malonyl CoA rises prior to any significant rise in consumption rate of malonyl CoA. Typically, the intracellular malonyl CoA in cells of the organism rises within 3 hours of administration, and intracellular malonyl CoA may be expected to rise prior to growth inhibition of the cells.

In a preferred mode of the method of this invention, the rise in intracellular malonyl CoA is correlated with reduced consumption of malonyl CoA. For example, the rise in intracellular malonyl CoA may be correlated with reduced intracellular activity of malonyl CoA decarboxylase (MCD) or reduced intracellular activity of fatty acid synthase; and optionally, the composition may comprise an inhibitor of MCD. In another preferred mode, the rise in intracellular malonyl CoA is correlated with increase synthesis of malonyl CoA and/or the rise in intracellular malonyl CoA is correlated with increased intracellular activity of acetyl-CoA carboxylase (ACC).

In one embodiment of the method of this invention, the composition comprises an agent selected from the group consisting of an activator of ACC, an activator of citrate synthase, an inhibitor of 5'-AMP-activated protein kinase (AMPK), and/or an inhibitor of acyl CoA synthase. In another mode of this invention, the composition comprises an inhibitor of carnitine palmitoyltransferase-1 (CPT-1), which may be etomoxir, preferably administered in combination with an agent from the proceeding group. In yet another embodiment of the method of this invention, a second chemotherapeutic agent is administered to the organism, said second chemotherapeutic agent being non-inhibitory to fatty acid synthesis.

Preferably, the method of this invention is used to treat organisms having, prior to administration of the composition, intracellular malonyl CoA level in tumor cells of at least 2-fold above normal malonyl CoA level in non-malignant cells. More preferably, the method is used to treat organisms where the fatty acid synthesis rate in some cells of the organism is at least 2-fold above that of normal cells prior to administration of the composition, and administration of the composition is cytotoxic to those cells. Preferably, the organism comprises tumor cells having elevated fatty acid synthesis rates and cell number of such tumor cells is reduced subsequent to administration of said composition. Upon treatment, preferably, the intracellular level of malonyl CoA is elevated and intracellular level of acetyl CoA, and free CoA are reduced relative to pretreatment levels.

In another embodiment, this invention provides a method for inhibiting growth of tumor cells in an organism comprising administering to said cells (a) an inhibitor of fatty acid synthesis in said cells; and (b) an inhibitor of fatty acid oxidation in said cells. Preferably, the inhibitor of fatty acid oxidation is administered in an amount which does not significantly inhibit CPT-2. More preferably, the inhibitor of fatty acid synthesis and the inhibitor of fatty acid oxidation are administered in amounts to achieve levels of inhibition which are at least about equal to or greater than the levels of the respective inhibitions observed for cytotoxic doses of cerulenin.

In yet another embodiment, this invention provides a screening method to assist in detecting compositions which are selectively cytotoxic to tumor cells comprising administering a target composition to a cell having an elevated intracellular malonyl CoA level, monitoring intracellular malonyl CoA in the cell subsequent to this administration, an abrupt increase in intracellular malonyl CoA being indicative of selective cytotoxicity. Preferably, this method further comprises comparing the pattern of intracellular malonyl CoA level changes in the presence and absence of TOFA, wherein reduced changes in malonyl CoA level in the presence of TOFA is indicative of selective cytotoxicity.

In still another embodiment, this invention provides a screening method to assist in detecting compositions which are growth inhibitory to tumor cells comprising administering a target composition to a tumor-derived cell line and monitoring CPT-1 activity in the cell subsequent to this administration, wherein a decrease in CPT-1 activity is indicative of growth inhibitory potential. Preferably, the method is carried out when the cell is permeabilized. Alternatively, the method further comprises monitoring said cell for apoptosis, and the monitoring for apoptosis may comprise a method selected from the group consisting of measuring mitochondrial transmembrane potential, staining with vital dyes, monitoring caspase activation in whole cells using Western blot, and measuring cytochrome C elaborated from mitochondria using Western blot.

### Brief Description of the Figures

Figure 1 shows the fatty acid synthesis pathway, and the effect of various fatty acid synthase inhibitors on fatty acid synthesis and tumor cell growth.
Figure 2 shows malonyl CoA levels under various conditions.
Figure 3 shows the results of clonogenic assays and apoptosis assays on breast cancer cells treated with various inhibitors.
Figure 4 shows various parameters in tumor cells and liver cells.

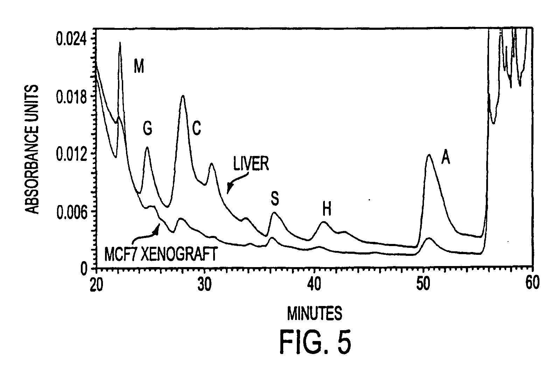

Figure 5 shows malonyl CoA levels in tumor cells and liver cells.
Figure 6 shows the pathway for cellular oxidation of fatty acids. CPT-1 regulates oxidation of fatty acids in the mitochondrion by controlling the passage of long chain acyl CoA derivatives such as palmitoyl CoA through the outer mitochondrial membrane into the mitochondrion, thus preventing the futile cycle of oxidizing endogenously synthesized fatty acids.
Figure 7 shows the effect of Etomoxir on growth of MCF-7 cells with and without C-75.
Figure 8 shows the effect of cerulenin on fatty acid oxidation in MCF-7 cells.
Figure 9 shows the effect of Etomoxir, TOFA and cerulenin on CPT-1 activity.
Figure 10 shows the effect of Etomoxir on fatty acid oxidation in MCF-7 cells.
Figure 11 shows the effect of Etomoxir on growth of MCF-7 cells.
Figure 12 shows the results of clonogenic assays with MCF-7 cells treated with both Etomoxir and TOFA.
Figure 13 shows the effect of Etomoxir and/or C-75 on growth of MCF-7 cells.

### Detailed Description of the Embodiments

If fatty acid starvation mediated the cytotoxic effects of cerulenin and C75, then any other FA synthesis inhibitor of similar potency should produce similar effects. To test this idea, the inventors compared the effects on cancer cells of inhibition of acetyl-CoA carboxylase (ACC, E.C. 6.4.1.2), the rate limiting enzyme of fatty acid synthesis, with the effects of FAS inhibitors. The inventors discovered that inhibition of FAS leads to high levels of malonyl-CoA which occurs within an hour of C75 treatment. These superphysiological levels of malonyl-CoA, rather than merely low levels of endogenously synthesized fatty acids, are responsible for breast cancer cell apoptosis. In addition, this is a novel pathway which leads to selective apoptosis of cancer cells.

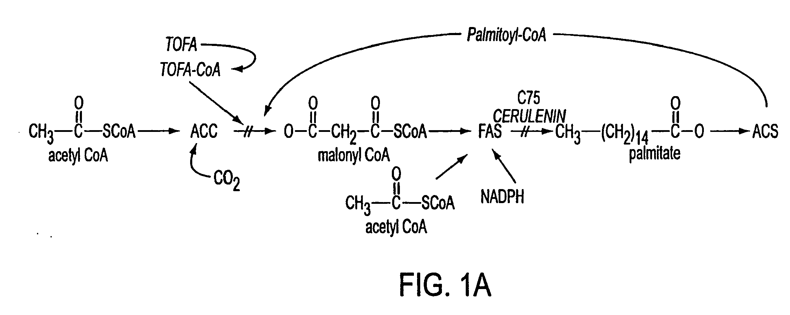

Figure 1A outlines the portion of the FA synthesis pathway containing the target enzymes of the inhibitors used in this study. Inhibition of fatty acid synthase results in high levels of malonyl-CoA that contribute to the cytotoxicity of against human breast cancer cells (ref). In addition to its role as a substrate for fatty acid synthesis, malonyl-CoA is a potent inhibitor of carnitine palmitoyltransferase-1 (CPT-1) the rate limiting enzyme of fatty acid oxidation. CPT-1 is an integral outer membrane protein of the mitochondrion that performs a trans-esterification of long chain fatty acyl CoA's to L-carnitine producing acylcarnitine. Acylcarnitine is transported across the mitochondrial membranes where it is esterified back to acyl-CoA by CPT-2. Physiologically, CPT-1 activity is regulated through inhibition by malonyl-CoA, a substrate of fatty acid synthesis. Malonyl-CoA is the enzymatic product of acetyl-CoA carboxylase (ACC, E.C. 6.4.1.2), the pace-setting enzyme for fatty acid synthesis. Cytoplasmic malonyl-CoA levels are higher during fatty acid synthesis due to increased activity of ACC. The high levels of malonyl-CoA, in turn, inhibits CPT-1, and blocks entry of long-chain acyl-CoA's into the mitochondrion. This prevents the futile cycle of simultaneous fatty acid synthesis and oxidation. In muscle, which is essentially devoid of FAS, ACC and malonyl-CoA regulate fatty acid oxidation, an important fuel source for cardiac and skeletal muscle.

TOFA (5-(tetradecyloxy)-2-furoic acid) is an allosteric inhibitor of acetyl-CoA carboxylase (ACC, E.C. 6.4.1.2), blocking the carboxylation of acetyl-CoA to malonyl-CoA. Once esterified to coenzyme-A, TOFA-CoA allosterically inhibits ACC with a mechanism similar to long chain acyl-CoA's, the physiological end-product inhibitors of ACC (Halvorson, D. L. and McCune, S. A. Inhibition of fatty acid synthesis in isolated adipocytes by 5-(tetradecyloxy)-2-furoic acid., Lipids. 19: 851-856, 1984). Both cerulenin (Funabashi, H., Kawaguchi, A., Tomoda, H., Omura, S., Okuda, S., and Iwasaki, S. Binding site of cerulenin in fatty acid synthetase., J. Biochem. 105: 751-755, 1989) and C75 (Pizer, et al., 1998) are inhibitors of FAS, preventing the condensation of malonyl-CoA and acetyl-CoA into fatty acids. Cerulenin is a suicide inhibitor, forming a covalent adduct with FAS (Moche, M., Schneider, G., Edwards, P., Dehesh, K., and Lindqvist, Y. Structure of the complex between the antibiotic cerulenin and its target, beta-ketoacyl carrier protein synthase., J Biol Chem. 274: 6031-6034, 1999), while C75 is likely a slow-binding inhibitor (Kuhajda, F.P., Pizer E.S., Mani, N.S., Pinn, M.L., Han W.F., Chrest F.J., and CA.T., Synthesis and anti-tumor activity of a novel inhibitor of fatty acid synthase, Proceeding of the American Association for Cancer Research, 40:121, 1999). Using TOFA, the inventors have achieved FA synthesis inhibition in human breast cancer cell lines comparable to inhibition by cerulenin or C75. Surprisingly, however, TOFA was essentially non-cytotoxic in clonogenic assays of human breast cancer cells. These data indicate that fatty acid starvation is not a major source of cytotoxicity to cancer cells in serum supplemented culture. An alternative effect of FAS inhibition (high levels of the substrate, malonyl-CoA, resulting specifically from inhibition of FAS) appears to mediate cytotoxicity of cerulenin and C75.

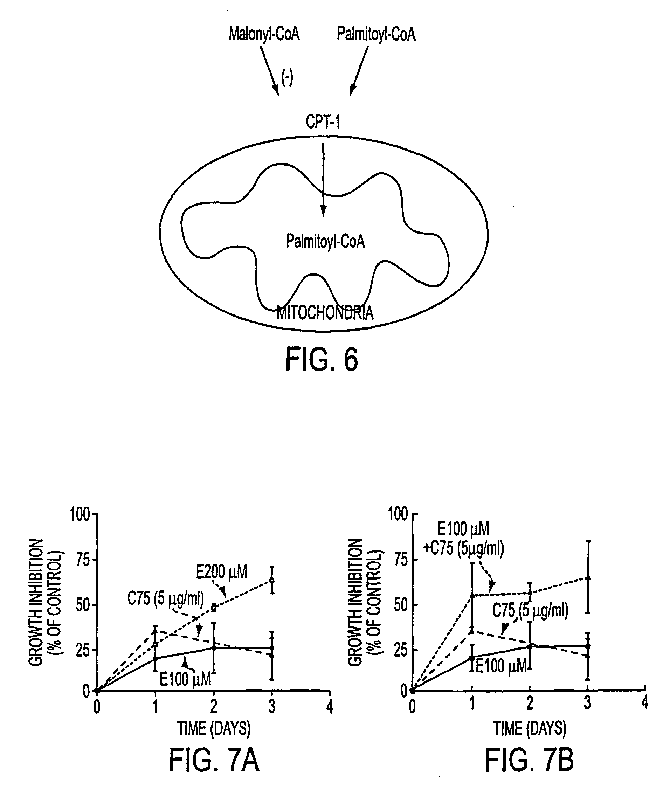

Malonyl-CoA, the enzymatic product of acetyl-CoA carboxylase (ACC, E.C. 6.4.1.2), is a key regulatory molecule in cellular metabolism. In addition to its role as a substrate in fatty acid synthesis, malonyl-CoA regulates β-oxidation of fatty acids through its interaction with carnitine palmitoyltransferase-1 (CPT-1) at the outer membrane of the mitochondria. Carnitine palmitoyltransferase (CPT-1) is the rate limiting enzyme of mitochondrial fatty acid oxidation (See Figure 6). It is an integral outer membrane protein of the mitochondrion that performs a *trans*-esterification of long chain fatty acyl CoA's to L-carnitine producing acylcarnitine. Acylcarnitine is transported across the mitochondrial membranes where it is esterified back to acyl-CoA by CPT-2.

Many types of cancer cells have high levels of fatty acid synthesis. As expected, cells with high levels of fatty acid synthesis have high steady state levels of malonyl-CoA, at least six times the levels in normal cells (see Example 6). Treatment of tumor cells with inhibitors of FAS will selectively and abruptly raise malonyl-CoA levels to superphysiological levels in cancer cells. This maneuver raises malonyl-CoA levels by both blocking utilization of malonyl-CoA as a substrate in fatty acid synthesis and concomitantly stimulating malonyl-CoA synthesis by relieving fatty acyl-CoA inhibition of ACC (Figure 1A). Since FAS is preferentially expressed in cancer cells, the malonyl-CoA elevation is largely restricted to tumors cells. This leads to cancer cell apoptosis and sparing of normal tissues as occurs in human cancer xenografts treated with FAS inhibitors (See Example 5).

CPT-1 has two isoforms, liver-type (L-CPT-1) and muscle-type (M-CPT-1) (Swanson, S. T., Foster, D. W., McGarry, J. D., and Brown, N. F. Roles of the N- and C-terminal domains of carnitine palmitoyltransferase I isoforms in malonyl-CoA sensitivity of the enzymes: insights from expression of chimaeric proteins and mutation of conserved histidine residues., Biochem . J. 335: 513-519, 1998). These isoforms have widely different kinetic properties in their *K*_{*m*} for carnitine (500 µM for M-CPT-1 and ∼30 µM for L-CPT-1) and sensitivity to malonyl-CoA inhibition (M-CPT-1 is 100-fold more sensitive, *K*_{*l*}= 0.07 µM versus 7 µM). While the regulatory site of malonyl-CoA resides in the N-terminal region, the exact binding site has not been elucidated (Swanson, et al., 1998). Importantly, etomoxir, a covalent inhibitor of CPT-1 that is used herein as an exemplary CPT-1 inhibitor, binds at a site different than that of malonyl-CoA.

CPT-1 has not been studied in human cancer cells. Hence, the isoform expressed in human cancer cells is unknown. Conceptually, the liver isoform should be expressed in tumors of epithelial differentiation which includes all carcinomas, while the muscle isoform would be expressed in non-epithelial tumors such as sarcomas. However, studies of ACC liver and muscle isoforms have found that either or both isoforms can be expressed in human breast cancer cells (Witters, L., Widmer, J., King, A., Fassihi, K., and Kuhajda, F. Identification of human acetyl-CoA carboxylase isozymes in tissue and in breast cancer cells., International Journal of Biochemistry. 26: 589-594, 1994). Similarly, human carcinoma cells may have the ability to express either or both CPT-1 isoforms.

Recently, inhibition of CPT-1 was shown to sensitize cells to fatty acid induced apoptosis (Paumen, M. B., Ishida, Y., Muramatsu, M., Yamamoto, M., and Honjo, T. Inhibition of carnitine palmitoyltransferase I augments sphingolipid synthesis and palmitate-induced apoptosis., J. Biol. Chem. 272: 3324-3329, 1997). Moreover, increased malonyl-CoA levels induced by the inhibition of fatty acid synthase (FAS) are cytotoxic to human cancer cells (see Examples 4 and 5). Taken together, these data suggest that human cancer cells are susceptible to induction of apoptosis via alterations in fatty acid metabolism. CPT-1 has also been shown to interact directly with BCL-2, the anti-apoptosis protein, at the outer mitochondrial membrane (Paumen, M. B., Ishisa, Y., Han, H., Muramatsu, M., Eguchi, Y., Tsujimoto, Y., and Honjo, T. Direct interaction of the mitochondrial membrane protein carnitine palmitoyltransferase I with Bcl-2, Biochem Biophys Res Commun. 231: 523-525, 1997). Potentially, the interaction of CPT-1 with BCL-2 may provide a down-stream mechanism leading to apoptosis by modulating the anti-apoptotic effects of BCL-2.

In addition to its role as a substrate for FAS, malonyl-CoA acts at the outer mitochondrial membrane to regulate fatty acid oxidation by inhibition of carnitine palmitoyltransferase 1 (CPT-1). Inhibition of CPT-1 has been shown to sensitize cells to fatty acid induced apoptosis; CPT-1 may also interact directly with BCL-2, the anti-apoptosis protein, at the mitochondria. FAS inhibition leads to high levels of malonyl-CoA inhibiting CPT-1 which induces cancer cell apoptosis. Since most proliferating and non-proliferating normal cells do not have high levels of FAS, they will not be affected by this therapeutic strategy.

Malonyl CoA levels may be manipulated using a variety of methods and target enzymes. The Examples demonstrate elevation of malonyl CoA levels through reduced utilization and simultaneous enhanced production. Acute increase in malonyl CoA levels lead to the selective destruction of cancer cells via apoptosis leaving normal cells unaffected. Methods for inducing apoptosis according to this invention fall into two broad categories: direct induction of acute increase in malonyl-CoA (e.g., by inhibiting FAS) and use of combination therapy to inhibit both fatty acid oxidation and fatty acid synthesis (e.g., through a non-FAS inhibitory mode). This therapeutic strategy identifies potential new targets and strategies for cancer chemotherapy based upon alteration of fatty acid metabolism.

Fatty acid oxidation may be inhibited via CPT-1 inhibition directly by inhibitory agents, such as etomoxir. Specific inhibitors to CPT-1 isoforms may also be developed. Alternatively, one could manipulate carnitine levels to reduce CPT-1 activity by reducing its substrate. Also, one may reduce CPT-1 expression levels either through genetic manipulation or by reducing exogenous fatty acids. Example 7 below is an example of the method of directly inhibiting CPT-1 using etomoxir in human breast cancer cells.

Other strategies for inhibiting fatty acid synthesis and oxidation include any method to increase malonyl-CoA levels from increased synthesis, decreased degradation, or preferably both. Malonyl-CoA levels may be manipulated using a variety of methods and target enzymes. Examples 4-5 demonstrate elevation of malonyl-CoA levels through reduced utilization and simultaneous enhanced production. Acute increase in malonyl-CoA levels leads to the selective destruction of cancer cells via apoptosis leaving normal cells unaffected. Other examples demonstrate additional ways to cause cancer cell growth inhibition or death.

Preferably, manipulation of fatty acid metabolism according to this invention is accomplished by administering a composition (or multiple compositions) to an organism in need thereof. The composition administered to the organism will contain an agent having at least one biological effect on fatty acid metabolic pathways, for example by raising intracellular malonyl-CoA levels. Typically, the organism will be a mammal, such as a mouse, rat, rabbit, guinea pig, cat dog, horse, cow, sheep, goat, pig, or a primate, such as a chimpanzee, baboon, or preferably a human. Usually, the organism will contain neoplastic (malignant) cells. The method of this invention is directed to selectively affecting malignant cells, and having less effect (or more preferably no effect) on normal (non-malignant) cells.

The agent in the composition administered to the organism will preferably raise the intracellular malonyl CoA levels in at least a portion of the malignant cells in the organism. Preferably the malonyl CoA level will be raised at least 2-fold, more preferably at least 5-fold. Preferably, the agent will raise the intracellular malonyl-CoA concentration in the malignant cells to a level higher than the level in surrounding normal cells.

Suitable agents may raise the malonyl CoA level by any of a number of methods (see alternative mechanisms listed below). Preferred agents typically induce a sudden or abrupt rise in malonyl CoA level. In some embodiments, two or more agents are administered, and some or all of these agents may affect malonyl CoA level by a different mechanism. Alternatively, a combination of agents may be used to lower fatty acid synthesis and simultaneously lower fatty acid oxidation. Preferably, the levels of fatty acid synthesis and oxidation will be lowered to levels comparable to those achieved by cytotoxic treatment with cerulenin. Agents acting by any of the modes of the following list may be used in compositions and methods of this invention. Assays for the following activities are available in the literature, and determination of whether a particular agent exhibits one of these activities is within the skill in the art.

### Increasing malonyl-CoA production:

Acetyl-CoA carboxylase (ACC) effectors: Agents which increase ACC activity, reduce ACC inhibition, or increase the mass of active ACC enzyme will lead to increased levels of malonyl-CoA.

5'-AMP protein kinase effectors: 5'-AMP protein kinase inhibits ACC by phosphorylation leading to acute reduction of malonyl-CoA. Inhibitors of this kinase would lead to acutely increased levels of malonyl-CoA by releasing inhibition of ACC.

Citrate synthase effectors: Increasing mitochondrial citrate would provide substrate for fatty acid synthesis, and citrate also acts as a "feed-forward" activator of ACC causing increase malonyl-CoA synthesis.

Acyl-CoA synthase effectors: Inhibition of acyl-CoA synthase would reduce cellular fatty acyl-CoA concentration releasing inhibition of ACC. This would result in increased ACC activity and malonyl-CoA levels.

### Decreasing malonyl-CoA utilization:

Malonyl-CoA decarboxylase (MCD) effectors: This enzyme catalyzes an ATP dependent decarboxylation of malonyl-CoA back to acetyl-CoA. Inhibition of MCD would acutely raise malonyl-CoA levels.

### Simultaneously decreased malonyl-CoA utilization and increased production:

Fatty acid synthase (FAS) effectors: Inhibition of FAS leads to decreased utilization of malonyl-CoA by blocking its incorporation into fatty acids. FAS inhibition also leads to reduced fatty acyl-CoA levels which will activate ACC. Exemplary FAS inhibitors may be obtained as described in U.S. Patent Nos. 5,759,837 and 5,981,575, incorporated herein by reference.

These strategies for modifying fatty acid metabolism, and especially for acutely increasing malonyl-CoA levels, may be used together or in concert with other drugs to enhance apoptosis of cancer cells. Preferably, at least one agent in the compositions of this invention raises the level of malonyl-CoA by a mechanism other than inhibiting FAS.

### ADMINISTRATION OF THE COMPONENTS

Therapeutic agents according to this invention are preferably formulated in pharmaceutical compositions containing the agent and a pharmaceutically acceptable carrier. The pharmaceutical composition may contain other components so long as the other components do not reduce the effectiveness of the agent according to this invention so much that the therapy is negated. Pharmaceutically acceptable carriers are well known, and one skilled in the pharmaceutical art can easily select carriers suitable for particular routes of administration (see e.g., Remington's Pharmaceutical Sciences, Mack Publishing Co., Easton, PA, 1985).

The pharmaceutical compositions containing any of the agents of this invention may be administered by parenteral (subcutaneously, intramuscularly, intravenously, intraperitoneally, intrapleurally, intravesicularly or intrathecally), topical, oral, rectal, or nasal route, as necessitated by choice of drug. The concentrations of the active agent in pharmaceutically acceptable carriers may range from 0.01 mM to 1 M or higher, so long as the concentration does not exceed an acceptable level of toxicity at the point of administration.

Dose and duration of therapy will depend on a variety of factors, including the therapeutic index of the drugs, disease type, patient age, patient weight, and tolerance of toxicity. Dose will generally be chosen to achieve serum concentrations from about 0.1 µg/ml to about 100 µg/ml. Preferably, initial dose levels will be selected based on their ability to achieve ambient concentrations shown to be effective in *in-vitro* models, such as those described herein, and *in-vivo* models and in clinical trials, up to maximum tolerated levels. Standard clinical procedure prefers that chemotherapy be tailored to the individual patient and the systemic concentration of the chemotherapeutic agent be monitored regularly. The dose of a particular drug and duration of therapy for a particular patient can be determined by the skilled clinician using standard pharmacological approaches in view of the above factors. The response to treatment may be monitored by analysis of blood or body fluid levels of the agent according to this invention, measurement of activity if the agent or its levels in relevant tissues or monitoring disease state in the patient. The skilled clinician will adjust the dose and duration of therapy based on the response to treatment revealed by these measurements.

### EXAMPLES

In order to facilitate a more complete understanding of the invention, a number of Examples are provided below. However, the scope of the invention is not limited to specific embodiments disclosed in these Examples, which are for purposes of illustration only.

### Example 1. Inhibition of FAS in cells in vitro

TOFA, Cerulenin, and C75 all inhibited fatty acid synthesis in human breast cancer cells. The human breast cancer cell lines, SKBR3 and MCF7 were maintained in RPMI with 10% fetal bovine serum. Cells were screened periodically for Mycoplasma contamination (Gen-probe). All inhibitors were added as stock 5 mg/ml solutions in DMSO. For fatty acid synthesis activity determinations, 5x10⁴ cells/well in 24 well plates were pulse labeled with [U-¹⁴C]-acetate after exposure to drug, and lipids were extracted and quantified as described previously (Pizer, et al., 1988). For MCF7 cells, pathway activity was determined after 2 hours of inhibitor exposure. SKBR3 cells demonstrated slower response to FAS inhibitors, possibly because of their extremely high FAS content, so pathway activity was determined after 6 hours of inhibitor exposure.

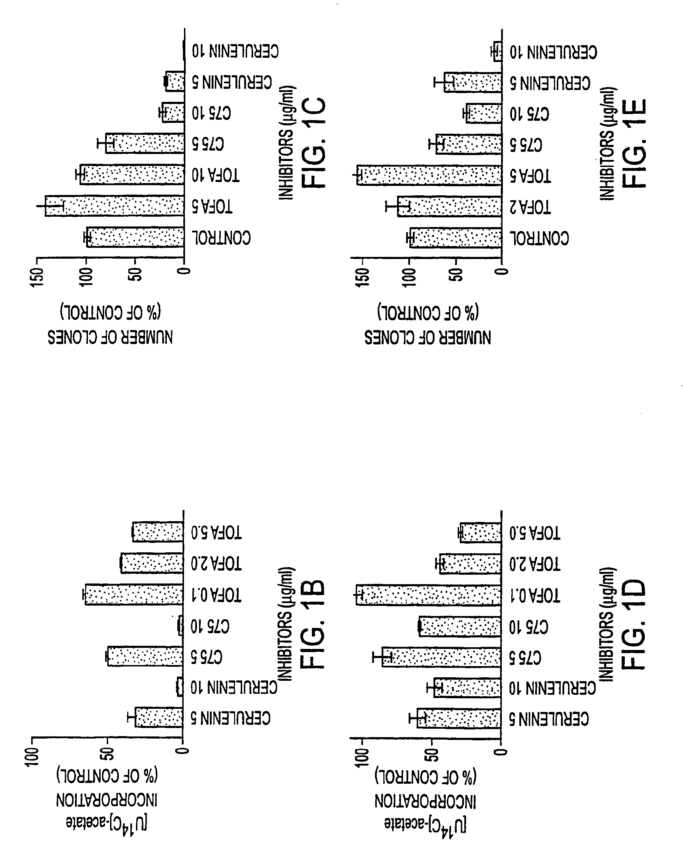

In standard pulse labeling experiments in which breast cancer cell lines, SKBR3 and MCF7 were labeled for 2 hours after exposure to FA synthesis inhibitors, TOFA, C75, and cerulenin all inhibited [U¹⁴C-acetate] incorporation into lipids to a similar extent (Figure 1B and D). In numerous similar experiments (not shown), TOFA maximally inhibited FA synthesis in the 1 to 5µg/ml dose range in all cell lines tested, and cerulenin and C75 maximally inhibited FA synthesis in the range of 10µg/ml.

### Example 2. Effect of the same inhibitors on cell growth

TOFA, Cerulenin, and C75 all inhibited fatty acid synthesis in human breast cancer cells, but showed differential cytotoxicity. Cells and inhibitors were as described for Example 1. For clonogenic assays, 4x10⁵ cells were plated in 25 cm³ flasks with inhibitors added for 6 hours in concentrations listed. Equal numbers of treated cells and controls were plated in 60mm dishes. Clones were stained and counted after 7 to 10 days.

Although all inhibitors reduced FA synthesis to a similar degree, TOFA was non-toxic or stimulatory to the cancer cell growth in the dose range for ACC inhibition, as measured by clonogenic assays, while cerulenin and C75 were significantly cytotoxic in the dose range for FAS inhibition (Figure 1C and E). The profound difference between the cytotoxic effects of ACC and FAS inhibition demonstrate that the acute reduction of fatty acid production *per se* is not the major source of cell injury after FAS inhibition.

### Example 3. Measurement of malonyl-CoA.

The most obvious difference in the expected results of inhibiting these two enzymes was that malonyl-CoA levels should fall after ACC inhibition, but should increase after FAS inhibition. Although not previously investigated in eukaryotes, recent data in *E. coli* have demonstrated elevated levels of malonyl-CoA resulting from exposure to cerulenin (Chohnan, et al., 1997, "Changes in the size and composition of intracellular pools of non-esterified coenzyme A and coenzyme A thioesters in aerobic and facultatively anaerobic bacteria," Applied and Environmental Microbiology, 63:555-560). Malonyl-CoA levels were measured in cells subjected to FAS inhibition and to inhibition by TOFA under conditions described in Example 2.

Malonyl-CoA levels were measured in MCF-7 cells using the HPLC method of Corkey, et al. (1988, "Analysis of acyl-coenzyme A esters in biological samples,"Methods in Enzymology, 166:55-70). Briefly, 2.5 x 10⁵ cells/well in 24 well plates were subjected to 1.2 ml of 10% TCA at 4° C after various drug treatments. The pellet mass was recorded and the supernatant was washed 6 times with 1.2 ml of ether and reduced to dryness using vacuum centrifugation at 25° C. Coenzyme-A esters were separated and quantitated using reversed phase HPLC on a 5 µ Supelco C18 column with a Waters HPLC system running Millenium³² software monitoring 254nm as the maximum absorbance for coenzyme-A. The following gradients and buffers were utilized: Buffer A: 0.1 M potassium phosphate, pH 5.0, Buffer B: 0.1 M potassium phosphate, pH 5.0, with 40% acetonitrile. Following a 20 min. isocratic run with 92% A, 8% B at 0.4 ml/min, flow was increased to 0.8 ml/min over one minute whereupon a linear gradient to 10% B was run until 24 min. then held at 10% B until 50 min. where a linear gradient was run to 100% B at 55 min., completing at 60 min. The following coenzyme-A esters (Sigma) were run as standards: malonyl-CoA, acetyl-CoA, glutathione-CoA, succinyl-CoA, HMG-CoA, and free CoA. Samples and standards were dissolved in 50 µl of buffer A. Coenzyme-A esters eluted sequentially as follows: malonyl-CoA, glutathione-CoA, free CoA, succinyl-CoA, HMG-CoA, and acetyl-CoA. Quantitation of coenzyme-A esters was performed by the Millenium³² software.

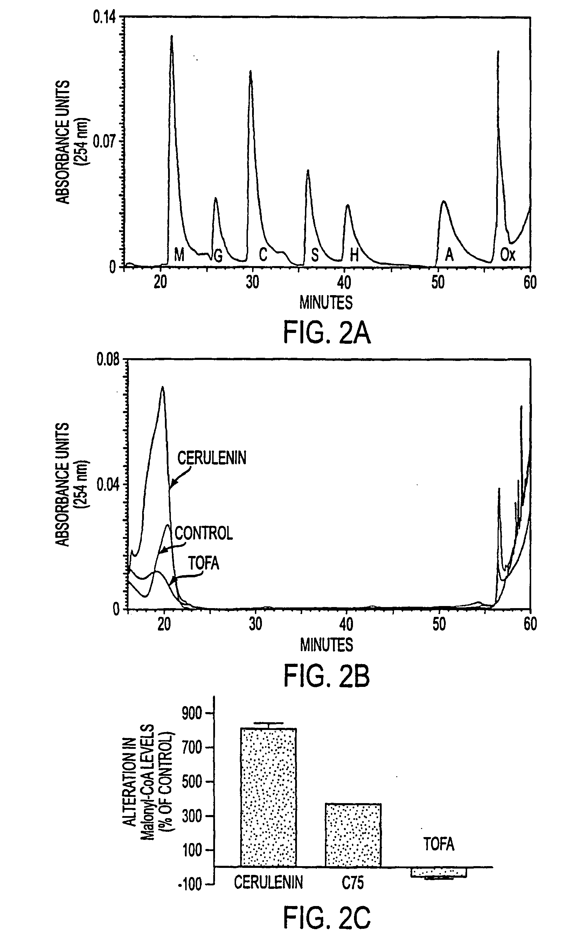

Direct measurement of coenzyme-A derivatives in MCF-7 cells by reversed phase HPLC of acid soluble extracts from drug treated cells confirmed that both cerulenin and C75 caused a rapid increase in malonyl-CoA levels while TOFA reduced malonyl-CoA levels. Figure 2A is a representative chromatograph demonstrating the separation and identification of coenzyme-A derivatives important in cellular metabolism. Malonyl-CoA is the first of these to elute, with a column retention time of 19-22 minutes. The overlay of chromatographs in Figure 2B shows that cerulenin treatment lead to a marked increase in malonyl-CoA over the control while TOFA caused a significant reduction. The chemical identity of the malonyl-CoA was independently confirmed by spiking samples with standards (not shown).

Malonyl-CoA levels were markedly increased with FAS inhibition and reduced by TOFA. Analysis of multiple experiments in Figure 2C demonstrated that following a 1 hour exposure to cerulenin or C75 at 10 µg/ml, malonyl-CoA levels increased by 930% and 370% respectively, over controls, while TOFA treatment (20 µg/ml) led to a 60% reduction of malonyl-CoA levels. The concentration of TOFA required for maximal reduction of malonyl-CoA levels was 4 fold higher than the dose for pathway inhibition in Figure 1B and D. However, optimal cultures for extraction of CoA derivatives had 5 fold higher cell density than the cultures used in the other biochemical and viability assays presented.

The remarkable increase in malonyl-CoA after FAS inhibition can be attributed in part to the release of long-chain fatty acyl-CoA inhibition of ACC leading to an increase in ACC activity (Figure 1A). Moreover, the cerulenin-induced increase in malonyl-CoA levels occurred within 30 minutes of treatment (930 +/-15% increase over control, not shown), within the time frame of FA synthesis inhibition, and well before the onset of DNA synthesis inhibition or early apoptotic events Thus, high levels of malonyl-CoA were a characteristic effect of FAS inhibitors and temporally preceded the other cellular responses, including apoptosis.

The levels of cerulenin or C75 which induce high levels of malonyl-CoA are cytotoxic to human breast cancer cells as measured by clonogenic assays and flow-cytometric analysis of apoptosis using merocyanin 450 staining. FAS inhibition causes high malonyl-CoA levels by inhibiting its consumption through FAS inhibition, with concomitant stimulation of synthesis by relieving the inhibitory effect of long-chain acyl-CoA's upon ACC activity (Figure 2).

### Example 4. TOFA rescue of FAS inhibition

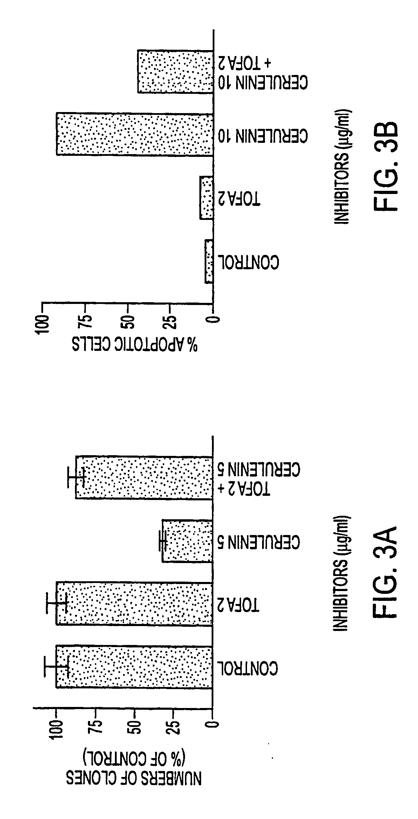

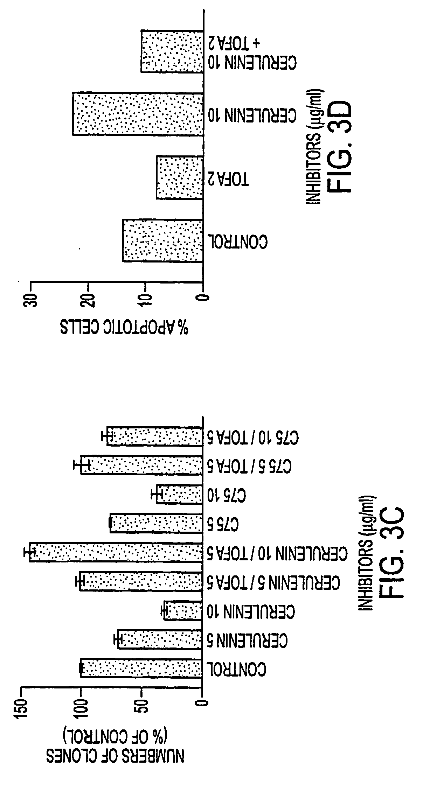

TOFA rescue of FAS inhibition demonstrates that high levels of malonyl-CoA are responsible for cancer cell cytotoxicity. If the elevated levels of malonyl-CoA resulting from FAS inhibition were responsible for cytotoxicity, then it should be possible to rescue cells from FAS inhibition by reducing malonyl-CoA accumulation with TOFA. Co-administration of TOFA and cerulenin to SKBR3 cells (Figure 3A) abrogated the cytotoxic effect of cerulenin alone in clonogenic assays performed as described in Example 2. In MCF7 cells (Figure 3C), TOFA produced a modest rescue of both cerulenin and C75 under similar experimental conditions.

Representative flow cytometric analyses of SKBR3 cells (Figure 3B) and MCF7 (Figure 3D) substantiated these findings, since TOFA rescued cells from cerulenin induced apoptosis. Apoptosis was measured by multiparameter flow cytometry using a FACStar^{Plus} flow cytometer equipped with argon and krypton lasers (Becton Dickinson). Apoptosis was quantified using merocyanine 540 staining (Sigma), which detects altered plasma membrane phospholipid packing that occurs early in apoptosis, added directly to cells from culture (Pizer, et al., 1998; Mower, et al., 1994, "Decreased membrane pospholipid packing and decreased cell size precede DNA cleavage in mature mouse B cell apoptosis, J. Immunol., 152:4832-4842). In some experiments, chromatin conformational changes of apoptosis were simultaneously measured as decreased staining with LDS-751 (Exciton) (Frey, et al., 1995, "Nucleic acid dyes for detection of apoptosis in live cells," Cytometry, 21:265-274). Merocyanine 540 [10µg/ml] was added as a 1 mg/ml stock in water. Cells were stained with LDS-751 at a final concentration of 100nM from a 1mM stock in DMSO. The merocyanine 540-positive cells were marked by an increase in red fluorescence, collected at 575 +/- 20 nm, 0.5 to 2 logs over merocyanine 540-negative cells. Similarly, the LDS-751 dim cells demonstrated a reduction in fluorescence of 0.5 to 1.5 logs relative to normal cells, collected at 660 nm with a DF20 band pass filter. Data were collected and analyzed using CellQuest software (Becton Dickinson).

In these experiments, all LDS-751 dim cells were merocyanine 540 bright, however a population of merocyanine 540 bright cells were detected that were not yet LDS-751 dim. All merocyanine 540 bright cells were classified as apoptotic. These experiments also confirmed the differential cytotoxicity between TOFA (<5% increase in apoptosis; no reduction in clonogenicity) compared to cerulenin (>85% apoptosis; 70% reduction in clonogenicity). Taken together, these studies show that high malonyl-CoA levels play a role in the cytotoxic effect of FAS inhibitors on cancer cells.

### Example 5. Effect of FAS inhibitors on tumor cell growth in vivo

To determine if the effects of FAS inhibition seen in vitro would translate to an in vivo setting requiring systemic activity, C75 was tested against subcutaneous MCF-7 xenografts in athymic nude mice, to quantitate effects on FA synthesis and the growth of established solid tumor. Previous studies have demonstrated local efficacy of cerulenin against a human cancer xenograft (Pizer, et al., 1996, "Inhibition of fatty acid synthesis delays disease progression in a xenograft model of ovarian cancer," Cancer Res., 56: 1189-1193), but were limited by the failure of cerulenin to act systemically. The similar responses of breast cancer cells to cerulenin and C75 in vitro suggested that C75 might be effective in vivo against xenografted breast cancer cells.

Subcutaneous flank xenografts of the human breast cancer cell line, MCF-7 in nu/nu female mice (Harlan) were used to study the anti-tumor effects of C75 *in vivo*. All animal experiments complied with institutional animal care guidelines. All mice received a 90-day slow-release subcutaneous estrogen pellet (Innovative Research) in the anterior flank 7 days before tumor inoculation. 10⁷ MCF-7 cells were xenografted from culture in DMEM supplemented with 10% FBS and insulin 10 µg/ml.

Treatment began when measurable tumors developed about 10 days after inoculation. Eleven mice (divided among two separate experiments of 5 and 6 mice each) were treated intraperitoneally with weekly doses of C75 at 30 mg/kg in 0.1 ml RPMI. Dosing was based on a single dose LD₁₀ determination of 40 mg/kg in BALB/c mice; 30 mg/kg has been well tolerated in outbred nude mice. Eleven control mice (divided in the same way as the treatment groups) received RPMI alone. Tumor volume was measured with calipers in three dimensions. Experiment was terminated when controls reached the surrogate endpoint.

In a parallel experiment to determine fatty acid synthesis activity in treated and control tumors, a group of MCF-7 xenografted mice were treated with C75 or vehicle at above doses and sacrificed after 3 hours. Tumor and liver tissue were *ex vivo* labeled with [U¹⁴⁻C] acetate, lipids were extracted and counted as described (Pizer, et al, 1996).

In an additional parallel experiment to histologically examine treated and control tumors, 6 C75 treated and 6 vehicle control mice were sacrificed 6 hours after treatment. Tumor and normal tissues were fixed in neutral-buffered formalin, processed for routine histology, and immunohistochemistry for FAS was performed. Immunohistochemistry for FAS was performed on the MCF-7 xenografts using a mouse monoclonal anti-FAS antibody (Alo, et al., 1996) at 1:2000 on the Dako Immunostainer using the LSAB2 detection kit.

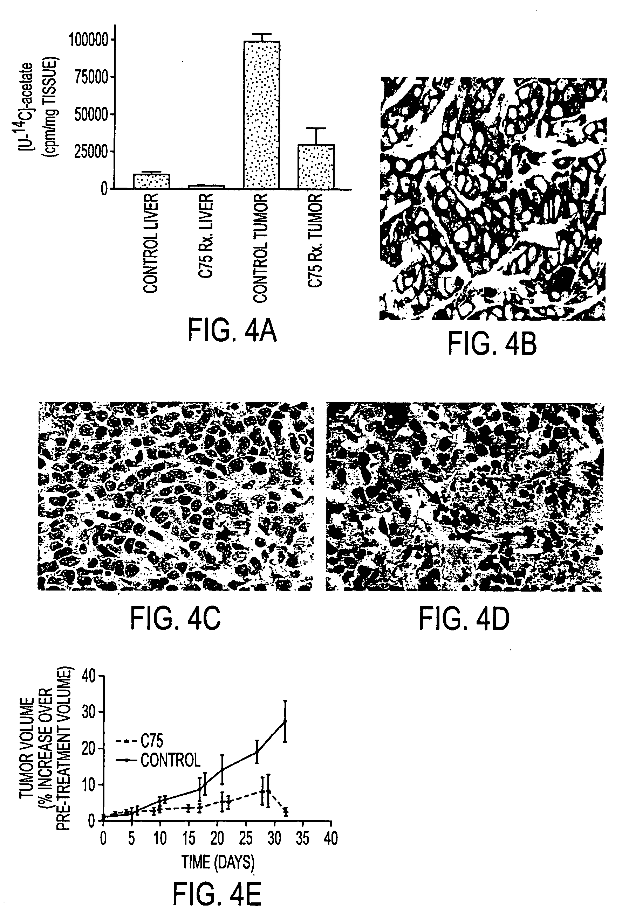

Fatty acid synthesis pathway activity in tissues of xenografted mice was determined by ex vivo pulse labeling with [U¹⁴C]-acetate. The tumor xenografts had 10-fold higher FA synthesis activity than liver, highlighting the difference in pathway activity between benign and malignant tissues (Figure 4A). FAS expression in the MCF-7 xenograft paralleled the high level of FA synthesis activity (Figure 4B). Intraperitoneal injections of C75 at 30 mg/kg reduced fatty acid synthesis in ex vivo labeled liver by 76% and in the MCF-7 xenografts by 70% within 3 hours (Figure 4A). These changes in FA synthesis preceded histological evidence of cytotoxicity in the xenograft, which became evident 6 hours after treatment (Figures 4 C and 4D). The C75 treated xenografts showed numerous apoptotic bodies throughout the tumor tissue, which were not seen in vehicle treated tumors. Histological analysis of liver and other host tissues following C75 treatment showed no evidence of any short or long term toxicity (not shown).

C75 treatment of the xenografts leads to cytotoxicity and reduction in tumor growth without injury to normal tissues. Tumor histology 6 hours following a 30 mg/kg dose of C75 demonstrates significant cytotoxicity compared to control tumor (Figures 4 C and 4D, attached preprint). Note the evidence of apoptotic bodies in the C75 treated xenograft while examination of liver and other organs show no evidence of tissue injury (data not shown). Weekly intraperitoneal C75 treatment retarded the growth of established subcutaneous MCF-7 tumors compared to vehicle controls, demonstrating a systemic anti-tumor effect (Figure 4E). After 32 days of weekly treatments, there was a greater than eight-fold difference in tumor growth in the treatment group compared to vehicle controls. Similar to cerulenin, transient reversible weight loss was the only toxicity noted (Pizer, et al., 1996).

The systemic pharmacologic activity of C75 provided the first analysis of the outcome of systemic FAS inhibitor treatment. The significant anti-tumor effect of C75 on a human breast cancer xenograft in the setting of physiological levels of ambient fatty acids was similar to the *in vitro* result in serum supplemented culture, and was consistent with a cytotoxic mechanism independent of fatty acid starvation.

### Example 6. Human cancer cells have high steady state levels of malonyl-CoA in vivo.

The result in Example 5 suggested that malonyl-CoA accumulation may not be a significant problem in normal tissues, possibly because FA synthesis pathway activity is normally low, even in lipogenic organs such as the liver. It is of further interest that, while malonyl-CoA was the predominant low molecular weight CoA conjugate detected in breast cancer cells in these experiments, other studies have reported predominantly succinyl-CoA and acetyl-CoA in cultured hepatocytes (Corkey, 1988). The high level of malonyl-CoA in the tumor tissues reflects the high level of fatty acid synthesis in the tumor cells compared to liver (Pizer, et al., 1996).

Using the MCF7 human breast cancer xenograft model of Example 5, malonyl-CoA levels were measured in the tumor xenograft and liver from the same animal using high-performance liquid chromatography. Figure 3 below shows high levels of malonyl-CoA in the tumor tissue compared to the liver. In addition, the distribution of other CoA derivatives are markedly altered. For example, while liver has about 10 fold less malonyl-CoA compared to the xenograft, it has about 10 fold higher levels of acetyl-CoA, and higher levels of other CoA derivatives, particularly succinyl-CoA. Differences in CoA derivative profiles may be indicative of larger differences in energy metabolism between cancer cells and hepatocytes.

### Example 7. Cell Growth Inhibition by CPT-1 Inhibitors

Carnitine palmitoyltransferase-1 is inhibited by etomoxir (Paumen, M. B., Ishida, Y., Muramatsu, M., Yamamoto, M., and Honjo, T. Inhibition of carnitine palmitoyltransferase I augments sphingolipid synthesis and palmitate-induced apoptosis., J. Biol. Chem. 272: 3324-3329, 1997; Ratheiser, K., Schneeweib, B., Waldhausl, W., Fasching, P., Korn, A., Nowotny, P., Rohac, M., and Wolf, H. P. O. Inhibition of etomoxir of carnitine palmitoyltransferse I reduces hepatic glucose production and plasma lipids in non-insulin-dependent diabetes mellitus., Metabolism. 40: 1185-1190, 1991).

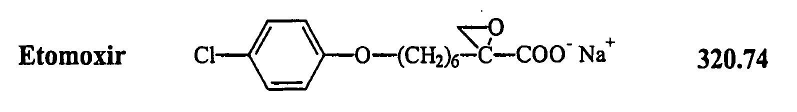

Figure 7A illustrates that etomoxir alone caused a significant growth inhibitory effect greater than C75nm. C75 indirectly inhibits CPT-1 by increasing malonyl-CoA. Figure 7B shows that etomoxir inhibition of growth of MCF-7 cells is additive with C75. In panel 7A, Etomoxir produces a dose dependent growth inhibition of MCF-7 cells over 72 h greater than that of C75 at 5 µg/ml. In panel 7B, etomoxir and C75 have a greater growth inhibitory effect than either alone. 5x10⁴ MCF-7 cells were plated in 24-well plates treated with inhibitors at the concentrations in the figure 18 h after plating. Cells are fixed with ethanol, stained with crystal violet, solubilized with SDS and read at 490. Importantly, the concentration of etomoxir is similar to that used in isolated hepatocytes to inhibit CPT-1; non-specific effects were identified at doses > 400 µM *in vitro* (Paumen, et al, 1997). When combined, etomoxir and C75 produced an additive growth inhibitory effect. Since malonyl-CoA and etomoxir are both CPT-1 inhibitors, and have different binding sites on CPT-1, the potentiating effect of etomoxir and C75 is not surprising. Etomoxir has been used to treat diabetes in humans without significant toxicity or weight loss (Ratheiser, et al., 1991). With this history, CPT-1 may provide a means to move this work more rapidly into the clinic.

### Example 8. Cerulenin Inhibits of Fatty Acid Oxidation.

Since increased levels of malonyl-CoA resulting from FAS inhibition have been shown to be cytotoxic in human breast cancer cells, we sought to determine if CPT-1 inhibition by malonyl-CoA also plays a role in the mechanism of cancer cell death.

MCF-7 human breast cancer cells were treated with cerulenin, a known FAS inhibitor, to determine if cerulenin causes decreased fatty acid oxidation at doses known to induce apoptosis in MCF-7 cells, but before the onset of actual apoptosis. Fatty acid oxidation was measured by trapping and counting the ¹⁴CO₂ released from the oxidation of [¹⁴C]palmitate in base.

1 x 10⁶ MCF-7 cells were plated in T-25 flasks in triplicate and incubated overnight at 37°C. The test compound (cerulenin) was then added as indicated diluted from 5 mg/ml stock in DMSO. After 2 hours, medium with drugs was removed and cells were preincubated for 30 minutes with 1.5 ml of the following buffer: 114 mM NaCl, 4.7 mM KCl, 1.2 mM KH₂PO₄, 1.2 mM MgSO₄, glucose 11 mM. After preincubation, 200 µl of assay buffer was added containing: 114 mM NaCl, 4.7 mM KCl, 1.2 mM KH₂PO₄, 1.2 mM MgSO₄, glucose 11 mM, 2.5 mM palmitate (containing with 100 µCi of [1-¹⁴C]palmitate) bound to albumin, 0.4 mM L-carnitine, and cells were incubated at 37°C for 2 hours. Following the incubation, 400 µl of benzothonium hydrochloride was added to the center well to collect released ¹⁴CO₂. Immediately, the reaction was stopped by adding 500 µl of 7% perchloric acid to the cells. The flasks with wells were then incubated for 2 hours at 37°C after which the benzothonium hydrochloride was removed and counted for ¹⁴C. Blanks were prepared by adding 500 µl of 7% perchloric acid to the cells prior to the incubation with the assay buffer for 2 hours.

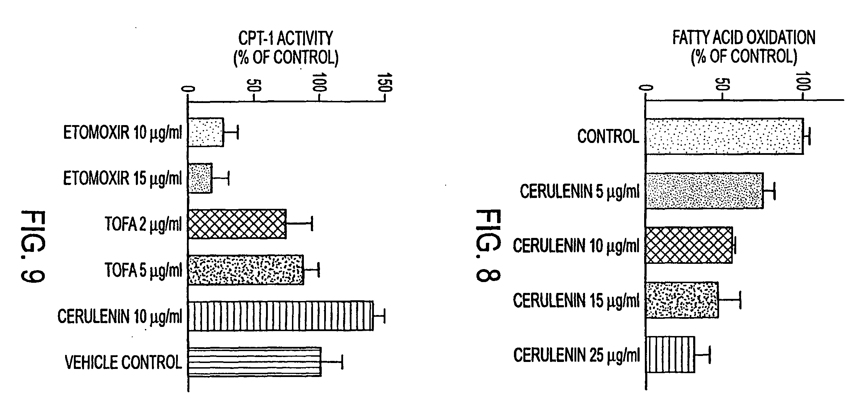

Figure 8 shows fatty acid oxidation in MCF-7 cells treated with cerulenin at the indicated doses for 2 hours, well before the onset of apoptosis in this system.

Cerulenin causes a dose-responsive inhibition of fatty acid oxidation in MCF-7 cells. At a dose of 10 µg/ml, which is known to cause nearly a nine-fold increase in malonyl-CoA and >50% reduction in fatty acid synthesis within 2 hours, cerulenin causes approximately a 50% reduction in fatty acid oxidation compared to control (p=0.0007; 2-tailed t-test)

### Example 9. Inhibition of Carnitine Paimitoyltransferase-1.

Cerulenin is known to induce an increase in malonyl CoA levels in cells when fatty acid synthase (FAS) is inhibited, and malonyl CoA is known to inhibit fatty acid oxidation through its effect on carnitine palmitoyltransferase-1 (CPT1). CPT-1 mediates the transfer of long-chain fatty acids into the mitochondria for β-oxidation. It performs a trans-esterification of long chain fatty acyl CoA's to L-carnitine producing acylcarnitine. Through this reaction, the water-soluble L-carnitine becomes organically soluble after esterification to the fatty acid. To test if the cerulenin-induced reduction in fatty acid oxidation is due to increased malonyl-CoA or through a direct inhibition of cerulenin on CPT-1, cerulenin was compared to other inhibitory compounds in a CPT-1 assay in MCF-7 cells.

**Carnitine Palmitoyltransferase-1 (CPT-1) Assay:** MCF-7 cells were plated in RPMI 1640 with 10% fetal bovine serum at 1x 10⁶ cells in six-well plates in triplicate. Following overnight incubation at 37°C, medium was removed and replaced with 700 µl of assay medium consisting of: 50 mM imidazole, 70 mM KCl, 80mM sucrose, 1 mM EGTA, 2 mM MgCl₂, 1 mM DTT, 1 mM KCN, 1 mM ATP, 0.1% fatty acid free bovine serum albumin, 70 µM palmitoyl-CoA, 0.25 µCi [methyl-¹⁴C]L-carnitine, 40 µg digitonin with or without 20 µM malonyl-CoA or other indicated inhibitors.

After incubation for 3 or 6 minutes at 37°C, the reaction was stopped by the addition of 500 µl of ice-cold 4 M perchloric acid. Cells were then harvested and centrifuged at 10,000 x g for 5 min. The pellet was washed with 500 µl ice cold perchloric acid and centrifuged again. The resulting pellet was resuspended in 800 µl dH₂O and extracted with 150 µl of butanol. The butanol phase was counted by liquid scintillation and represents the acylcarnitine derivative.

Figure 9 shows the effect of three compounds on CPT-1: Etomoxir (a known inhibitor of CPT-1), TOFA (known to inhibit fatty acid synthesis by inhibiting acetyl CoA carboxylase, an enzyme in the fatty acid synthesis pathway) and cerulenin.

Figure 9 shows that cerulenin does not inhibit CPT-1 directly in MCF-7 cells. In fact, at 10 µg/ml, cerulenin causes a slight, but not statistically significant increase in CPT-1 activity above vehicle control. Thus, the decrease in fatty acid oxidation induced by cerulenin is likely due to the concurrent increase in malonyl-CoA rather than from a direct effect of cerulenin on CPT-1.

### Example 10. Effect CPT-1 Inhibition on Cell Growth.

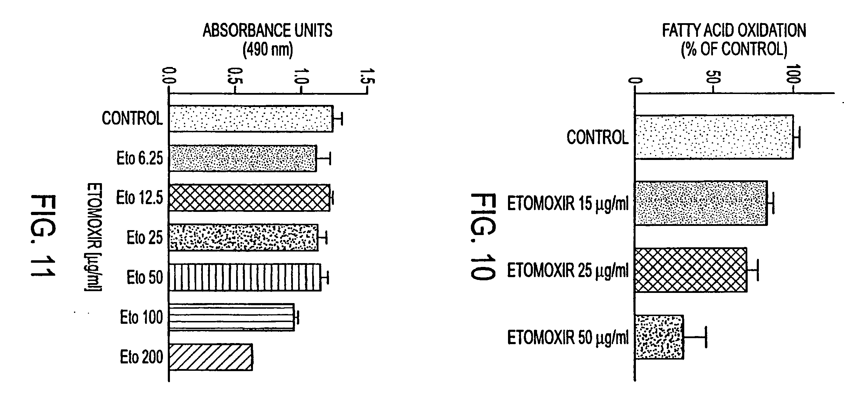

Since cerulenin causes an increase in malonyl-CoA and decreased fatty acid oxidation, tests were devised to see if CPT-1 inhibition was involved in triggering apoptosis. For that purpose, MCF-7 cells were treated with Etomoxir, a known direct inhibitor of CPT-1, and fatty acid oxidation by the cells was measured as described in Example 8. Figure 10. shows that Etomoxir causes inhibition of fatty acid oxidation in MCF-7 cells.

At a dose of 50 µg/ml fatty acid oxidation is decreased by >50% over control (p=0.012; 2-tailed t-test). (Figure 9 demonstrates that Etomoxir directly inhibits CPT-1, with a dose of 10 µg/ml causing a 75% reduction in CPT-1 activity, p=0.023; 2-tailed t-test.)

***Cell growth inhibition assay:*** Although Etomoxir is a potent inhibitor of CPT-1, when MCF-7 cells are treated with doses of Etomoxir known to inhibit CPT-1 and fatty acid oxidation, there is no significant growth inhibition or cytotoxicity. MCF-7 cells were plated in 24-well plates at 5x10⁴ cells per well in RPMI 1640 with 10% fetal bovine serum (Hyclone). After overnight incubation at 37°C, Etomoxir was added from stock 5 mg/ml solutions in DMSO. The final concentration of DMSO in the cultures was at or below 0.2%. After either 48 or 72 h, medium was removed, and wells were washed thrice with Hank's buffered saline. Wells were stained with crystal violet, then dried, and solubilized in 10% SDS. 100 µl aliquots were transferred to a 96-well plate and read on a Molecular Dynamics plate reader at 490 nm. Data are presented as absorbance units with error bars showing standard error of the mean. Statistics and graphing were performed in Prism 2.0 (Graph Pad).

Figure 11 shows the effect of Etomoxir on growth inhibition in MCF-7 cells.

Only the 200 µg/ml dose caused a significant reduction in growth (p=0.006, two-tailed t-test). Thus, CPT-1 inhibition alone is significantly growth inhibitory to human breast cancer cells.

During cerulenin treatment, however, CPT-1 is inhibited and fatty acid oxidation is reduced during fatty acid synthesis inhibition; this is a non-physiologic response. Physiologically, when fatty acid synthesis is reduced, malonyl-CoA levels fall, relieving the inhibition of CPT-1 causing an increase in fatty acid oxidation. Thus, it is possible that CPT-1 inhibition also induces cytotoxicity in the setting of fatty acid synthesis inhibition.

### Example 11. Cytotoxic Effect of CPT-1 Inhibition and Fatty Acid Synthesis Inhibition in Combination.

TOFA is an inhibitor of acetyl-CoA carboxylase (ACC), the rate limiting enzyme in fatty acid synthesis. TOFA inhibition of ACC causes a reduction in malonyl-CoA and subsequent inhibition of fatty acid synthesis. While both TOFA and cerulenin cause inhibition of fatty acid synthesis, cerulenin inhibits FAS that leads to an increase in malonyl-CoA while TOFA inhibits ACC which causes a decrease in malonyl-CoA.

In this Example, cells were treated with TOFA to inhibit fatty acid synthesis and Etomoxir to inhibit fatty acid oxidation. The effect of this combined inhibition on cytotoxicity was measured in a clonogenic assay.

**Clonogenic Assay:** After overnight incubation at 37°C, 1x10⁶ MCF-7 cells were exposed to drugs as indicated for 6 h, washed, detached by trypsin digestion, counted and plated at 1000 or 500 cells/ 60-mm plate in triplicate. Colonies were stained with crystal violet and counted 4-6 days after plating. Controls consisted of cells incubated with DMSO without drugs. Error bars represent standard error of the mean.

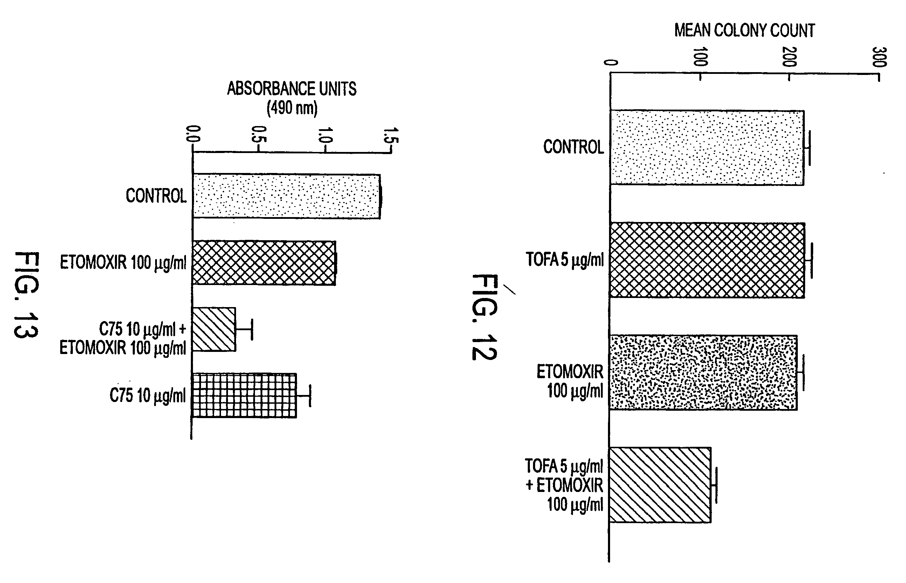

Figure 12 shows a clonogenic assay with MCF-7 cells treated with both Etomoxir and TOFA.

Treatment of the cells with TOFA at 5 µg/ml is not significantly cytotoxic; this is similar to our previously published studies (ref). Figure 9 also shows that TOFA does not cause CPT-1 inhibition, nor does TOFA cause significant changes in fatty acid oxidation (data not shown). Etomoxir treatment is also not significantly cytotoxic complementing the growth inhibition studies in Figure 11. However, the combination of TOFA and Etomoxir is significantly more cytotoxic than TOFA alone (p=0.004, two-tailed t-test) or Etomoxir alone (p=0.002, two-tailed t-test).

These data indicate that CPT-1 inhibition is toxic to cancer cells during fatty acid synthesis inhibition. Therefore. CPT-1 inhibitors could be used in conjunction with fatty acid synthesis inhibitors to increase anti-tumor response.

### Example 12. Additive effects on Cytotoxicity of a Fatty Acid Synthase Inhibitor and a CPT-1 Inhibitor.

In growth inhibition assays using the procedure describe in Example C, MCF-7 cells were treated with C75, an FAS inhibitor, alone or with Etomoxir and analyzed 48 hours after treatment. Both Etomoxir and C75 caused significant growth inhibition over control (p=0.0001, p=0.005, two-tailed t-test). Figure 13 below shows that etomoxir can also enhance the cytotoxic effect of FAS inhibition.

The combination of Etomoxir and C75 caused more significant growth inhibition than Etomoxir alone (p=0.004, two-tailed t-test) and a strong trend toward increased growth inhibition than C75 alone (p=0.054 two-tailed t-test). These data suggest that CPT-1 inhibition may also enhance the anti-tumor effect of FAS inhibitors.

For purposes of clarity of understanding, the foregoing invention has been described in some detail by way of illustration and example in conjunction with specific embodiments, although other aspects, advantages and modifications will be apparent to those skilled in the art to which the invention pertains. The foregoing description and examples are intended to illustrate, but not limit the scope of the invention. Modifications of the above-described modes for carrying out the invention that are apparent to persons of skill in medicine, biochemistry, pharmacology, and/or related fields are intended to be within the scope of the invention, which is limited only by the appended claims.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

## Claims

1. A method for inhibiting growth of tumor cells in an organism comprising administering to the organism a composition which causes a rise in intracellular malonyl CoA in tumor cells of said organism.

2. The method of claim 1, wherein intracellular malonyl CoA in cells of said organism rises abruptly.

3. The method of claim 1, wherein intracellular malonyl CoA rises prior to any significant rise in consumption rate of malonyl CoA.

4. The method of claim 1, wherein intracellular malonyl CoA in cells of said organism rises within 3 hours of said administration.

5. The method of claim 1, wherein intracellular malonyl CoA rises prior to growth inhibition of the cells.

6. The method of claim 1, wherein said rise in intracellular malonyl CoA is correlated with reduced consumption of malonyl CoA.

7. The method of claim 1, wherein said rise in intracellular malonyl CoA is correlated with reduced intracellular activity of malonyl CoA decarboxylase (MCD) or reduced intracellular activity of fatty acid synthase.

8. The method of claim 1, wherein said composition comprises an inhibitor of MCD.

9. The method of claim 1, wherein said rise in intracellular malonyl CoA is correlated with increase synthesis of malonyl CoA.

10. The method of claim 1, wherein said rise in intracellular malonyl CoA is correlated with increased intracellular activity of acetyl-CoA carboxylase (ACC).

11. The method of claim 1, wherein said composition comprises an agent selected from the group consisting of an activator of ACC, an activator of citrate synthase, an inhibitor of 5'-AMP-activated protein kinase (AMPK), and/or an inhibitor of acyl CoA synthase.

12. The method of claim 1, wherein a second chemotherapeutic agent is administered to the organism, said second chemotherapeutic agent being non-inhibitory to fatty acid synthesis.

13. The method of claim 1, wherein intracellular malonyl CoA level in tumor cells prior to administration of said composition is at least 2-fold above normal malonyl CoA level in non-malignant cells.

14. The method of claim 1, wherein intracellular level of malonyl CoA is elevated and intracellular level of acetyl CoA, and free CoA are reduced relative to pretreatment levels.

15. The method of claim 1, wherein fatty acid synthesis rate in some cells of said organism is at least 2-fold above normal prior to administration of said composition, and administration of said composition is cytotoxic to said cells.

16. The method of claim 1, wherein said organism comprises tumor cells having elevated fatty acid synthesis rates and cell number of said tumor cells is reduced subsequent to administration of said composition.

17. The method of claim 1, wherein said composition comprises an inhibitor of carnitine palmitoyltransferase-1 (CPT-1).

18. The method of claim 1, wherein said composition comprises etomoxir.

19. A method for inhibiting growth of tumor cells in an organism comprising administering to said cells
a) an inhibitor of fatty acid synthesis in said cells; and
b) an inhibitor of fatty acid oxidation in said cells.

20. The method of claim 19, wherein said inhibitor of fatty acid oxidation is administered in an amount which does not significantly inhibit CPT-2.

21. The method of claim 19, wherein said inhibitor of fatty acid synthesis and said inhibitor of fatty acid oxidation are administered in amounts to achieve similar levels of their respective inhibitions as observed for cytotoxic doses of cerulenin.

22. A screening method to assist in detecting compositions which are selectively cytotoxic to tumor cells comprising administering a target composition to a cell having an elevated intracellular malonyl CoA level, monitoring intracellular malonyl CoA in said cell subsequent to said administration, wherein an abrupt increase in intracellular malonyl CoA is indicative of selective cytotoxicity.

23. The method of claim 22, further comprising comparing pattern of intracellular malonyl CoA level changes in the presence and absence of TOFA, wherein reduced changes in malonyl CoA level in the presence of TOFA is indicative of selective cytotoxicity.

24. A screening method to assist in detecting compositions which are growth inhibitory to tumor cells comprising administering a target composition to a tumor-derived cell line, monitoring CPT-1 activity in said cell subsequent to said administration, wherein a decrease in CPT-1 activity is indicative of growth inhibitory potential.

25. The method of claim 24, wherein said cell is permeabilized.

26. The method of claim 24, further comprising monitoring said cell for apoptosis.

27. The method of claim 26, wherein monitoring for apoptosis comprises a method selected from the group consisting of measuring mitochondrial transmembrane potential, staining with vital dyes, monitoring caspase activation in whole cells using Western blot, and measuring cytochrome C elaborated from mitochondria using Western blot.
